# EUROPEAN PATENT SPECIFICATION

(11) **EP 1 222 953 B1**
(45) Date of publication and mention of the grant of the patent: **05.05.2010**
(21) Application number: 02075482.6
(22) Date of filing: 07.04.1997
(51) Int. Cl.: B01D 61/44, C07C 51/02, C07C 65/03

(54) **Process for the preparation of p-hydroxybenzoic acid**
Verfahren zur Herstellung von P-HYDROXYBENZOESÄURE
Procédé pour la preparation d' acide P-HYDROXYBENZOIQUE

(30) Priority: 08.04.1996 US 14998 P; 04.04.1997 US 832739
(43) Date of publication of application: 17.07.2002
(62) Divisional of application: 97920221.5
(73) Proprietor: E.I. DU PONT DE NEMOURS AND COMPANY, Wilmington, DE 19898 (US)
(72) Inventor: Citron, Joel David, Wilmington, DE 19810 (US); Samuels, Michael Robert, Wilmington, DE 19808 (US)
(74) Representative: Morf, Jan Stefan

(56) References cited:
- EP-A- 0 438 369
- GB-A- 1 198 087
- US-A- 4 093 528
- US-A- 4 381 229
- US-A- 4 828 993
- CHEMICAL ABSTRACTS, vol. 83, no. 4, 28 July 1975 (1975-07-28) Columbus, Ohio, US; abstract no. 34773, HAKUSHI, TADAO; DOHNO, REIZO; AZUMI, TAKATSUGU; TAKASHIMA, SHIRO: "Ion-exchange membranes. XXIV. Electrodialytic concentration of carboxylic acids using ion-exchange resins" XP002037077 & HIMEJI KOGYO DAIGAKU KENKYU HOKOKU, vol. 27a, 1974, pages 141-144,
- RAUCQ D ET AL: "PRODUCTION OF SULPHURIC ACID AND CAUSTIC SODA FROM SODIUM SULPHATE BY ELECTROMEMBRANE PROCESSES COMPARISON BETWEEN ELECTRO-ELECTRODIALYSIS AND ELECTRODIALYSIS ON BIPOLAR MEMBRANE" DESALINATION, vol. 91, no. 2, 1 April 1993 (1993-04-01), pages 163-175, XP000453350

## Description

### FIELD OF THE INVENTION

This invention also concerns a process for the isolation of p-hydroxybenzoic acid from its mono- or dialkali metal salts by electrodialyzing these salts. Alkali metals and their hydroxides may be completely and economically recycled in the process.

### BACKGROUND OF THE INVENTION

Aromatic hydroxycarboxylic acids and dicarboxylic acids are important items of commerce. For instance o-hydroxybenzoic acid (salicylic acid) is used as a chemical intermediate, for instance to make aspirin, while p-hydroxybenzoic acid (PHBA) is used to make parabens and is also used as a monomer in making polyesters, while dicarboxylic acids are important as monomers. Traditionally aromatic hydroxycarboxylic acids are manufactured using the Kolbe-Schmitt reaction, which is a reaction of an alkali metal salt of an aromatic hydroxy compound with carbon dioxide, usually under elevated temperature and pressure. The Kolbe-Schmitt reaction has been a standard procedure for the preparation of aromatic hydroxy acids for over 100 years, see for instance A. S. Lindsey, et al.. Chem. Rev.. vol. 57, p. 583-620 (1957) incorporated by reference herein. However, this process is complex and difficult to run. involving several manufacturing steps, which adds to the cost of the final product. Since the initial product of the carboxylation reaction is a dialkali metal salt of the aromatic hydroxycarboxylic acid, substantial cost is usually incurred for the use of compounds such as NaOH or KOH which are subsequently discarded (as sodium or potassium salts), since the free aromatic hydroxycarboxylic acid (or dicarboxylic acid) is usually isolated by reacting the dialkali metal salt with a strong acid. It is hence desirable to develop an improved Kolbe-Schmitt process for the manufacture of these compounds. Dicarboxylic acids are also sometimes available as their dialkali metal salts, and it is often desirable to convert these to the dicarboxylic acids themselves, and generate an alkali from the alkali metals present in the salts.

It is known that the salts of diacids or aromatic hydroxycarboxylic acids can be electrodialyzed to form the free dicarboxylic acid or aromatic hydroxycarboxylic acid and the alkali metal hydroxide. However, when one tries to completely electrodialyze these compounds to these final products, as one approaches complete electrolysis, the voltage increases and the current efficiency decreases rapidly and the process may become uneconomic. Therefore, it would be desirable to have another economical method for isolating the free aromatic hydroxycarboxylic acid or dicarboxylic acid from its dialkali metal salt, while at the same time being able to recycle the alkali metals in the process in an economical fashion.

Japanese Patent Application 40-11492 describes the electrodialysis of an alkali metal salt of terephthalic acid to terephthalic acid and an alkali metal hydroxide.

US 4, 093, 528 describes a process for the preparation of terephthalic acid which comprises electrolyzing disodium terephthalate in the anode compartment of an electrolytic can whereby terephthalic acid product is precipitated and subsequently separated.

Japanese Patent Application 64-9954 describes the electrodialysis of an alkali metal salt of hydroxybenzoic acid.

None of the above references describes a partial electrodialysis followed by a treatment with a strong acid to effect isolation of a dicarboxylic acid or an aromatic hydroxycarboxylic acid.

Electrodialysis of salts of organic compounds is in general known, and generally requires only relatively simple equipment. However, if one attempts to electrodialyze an aqueous solution of the mono- or dipotassium salt of PHBA, one finds that before free PHBA is obtained, the voltage required to effect electrolysis greatly increases and the electrolysis essentially stops (see Comparative Example 1). However it has now been found that if this electrodialysis is done at elevated temperatures, good results can be obtained.

### SUMMARY OF THE INVENTION

A process of this invention for the preparation of p-hydroxybenzoic acid from its alkali metal salt, comprises, electrodialyzing an aqueous solution of a first compound of the formula (OR¹CO₂)HₜM₂₋ₜ to produce a second compound of the formula (OR¹CO₂)H_{y}M_{2-y} and MOH, wherein:
R¹ is p-phenylene;
t is zero to 1.50;
M is an alkali metal cation; and
y is 1.95 to 2.00;
and provided that when y or t is about 1.0 or more, said electrodialysis is carried out at a temperature of 75°C or more.

### BRIEF DESCRIPTION OF THE DRAWINGS

Figure 1 is a graph showing data from Example 1 of electrolysis time vs. pH of the solution in the central compartment of the electrodialysis cell.
Figure 2 is a graph showing data from Comparative Example 1 of electrolysis time vs. pH of the solution in the central compartment of the electrodialysis cell.

### DETAILED DESCRIPTION OF THE INVENTION

The product of the process of the invention is p-hydroxybenzoic acid, containing up to 5 mole percent of the monopotassium salt (y=1.95). Obtaining complete electrodialysis to "pure" p-hydroxybenzoic acid may require an inordinate amount of electrical energy, so it may be more economical to leave a small amount of the monopotassium salt in the PHBA and purify the free compound as by crystallization. The monopotassium salt left in solution may be recycled back to the electrodialysis for recovery.

The starting material for the process is the corresponding mono- or dialkali metal salt of PHBA of the formula (OR¹CO₂)HₜM₂₋ₜ wherein t is zero to about 1.5, more preferably 0 to about 0.5, especially preferably less than about 0.1, and most preferably about 0.0. This PHBA salt is then electrolyzed so that the value of t is increased to y, y normally being greater than t. Usually there will be essentially only one alkali metal present, and potassium is preferred. Using the process described herein, an essentially closed loop process with respect to alkali metal (usually potassium) may be envisioned.

In the final product of the inventive process it is preferred that y is about 1.97 or more.

A three compartment cell may be utilized in the process which utilizes an alkali metal salt of PHBA. This starting material is fed to the center compartment, while alkali metal hydroxide will be generated in the cathode compartment. In the anodic compartment oxygen is generated, while in the center compartment the compound (OR¹CO₂)H_{y}M_{2-y} is made. Fresh solution of the PHBA alkali metal salt may be added (continuously or intermittently) to the center compartment, and solution of the center compartment removed (continuously or intermittently), at such a rate so that ''average" solute in the solution is (OR¹CO₂)H_{y}M_{2-y}, as defined herein.

In the process when y is about 1 or more, the electrodialysis is carried out above about 75°C. Thus two cells can be used in series if in the starting alkali metal salt of PHBA t is about 1.0 or less. In this instance the temperature in the first cell is not critical, but in the second cell, wherein y is about 1 or more, the temperature is about 75°C or more. Temperatures above the atmospheric boiling point of water in the solution may be used by placing the cell under elevated pressure, but the preferred upper temperature limit for this part of the electrodialysis is the boiling point of the aqueous solution at atmospheric pressure. The preferred minimum temperature is about 80°C, and it is more preferred that the minimum temperature is about 85°C.

In the present process the concentration of the alkali metal salt of PHBA in the aqueous solution that is electrodialyzed is not critical, but not so high that free PHBA will crystallize out in the three compartment cell. However, it is preferred that the concentration is high enough so that the solution will readily conduct electricity. It is also preferred that the solution concentration be relatively high so that isolation of the free PHBA after electrolysis is simplified. Isolation may be accomplished by cooling the solution and separating the crystallized PHBA. The filtrate containing some dissolved PHBA may be recycled back into the electrodialysis, i.e., "new" alkali metal salt may be dissolved in the filtrate and the solution electrodialyzed. A preferred concentration of alkali metal salt in solution is about 10 to about 35 percent by weight, more preferably about 15 to about 30 percent by weight, of free PHBA based on the total weight of water and free PHBA equivalent in the solution.

### EXAMPLE 1

The electrochemical cell used was an ElectroCell AB (S-184 00 Akersberga, Sweden) "Electro MP Cell". This is was configured as a three compartment cell using Nafion® N-417 (formerly commercially available from E. I. du Pont de Nemours and Company, Wilmington, DE, U.S.A.) membranes. This membrane was a perfluorosulfonic acid polymer with an equivalent weight of 1100 reinforced with a woven perfluoropolymer fabric. The nominal thickness of the membrane was about 0.25 mm, and it had conditioned resistance of 3.5-4.0 ohms-cm². Current similar offerings of Nafion® include Nafion® N-450 and Nafion® NE-424. The effective area of each of the anode and cathode was 0.01 m². The anode was a dimensionally stable (DSA) oxygen anode, and the cathode was stainless steel.

The PHBA solution was placed in a 2 L resin kettle with a lid and clamp. The kettle was heated on a hot plate and was equipped with a magnetic stirrer, pH meter electrode, thermometer, and process inlet and outlet lines. The outlet line also had a porous thermoplastic disc filter in it. The PHBA solution was passed through a glass vacuum trap which was wrapped with electrical heater tape and acted as an auxiliary heater. The PHBA was circulated to the center compartment of the electrolysis cell.

The catholyte was 1.5 L of 1N KOH solution which was pumped from a heated reservoir to the cathode compartment and then returned to the reservoir. The temperature of the catholyte was kept close to the PHBA solution temperature.

The anolyte was 50 mL of concentrated sulfuric acid diluted in 900 mL of distilled water. It was circulated by a pump from a reservoir through the anode compartment back to the reservoir. The anolyte had no separate heater.

A solution of the dipotassium salt of p-hydroxybenzoic acid (PHBA) was made by dissolving 120 g of PHBA and 114.6 g of KOH (pellets nominally containing 85 weight percent KOH, 15% water) in 400 mL of water. This was circulated in the center compartment of the cell, and the solution in all three compartments were separately circulated and heated to 90°C (during the electrolysis the catholyte was 88°C and the center compartment solution was 83°C at the start, and at 90°±1°C within 25 min after the start of the electrolysis). The electrolysis was started and continued so that the voltage was varied to maintain a constant current of 15 A (ampere). During the electrodialysis water was added as necessary to replace evaporative losses.

The voltages required vs. the time elapsed for selected times during the electrolysis are shown Table 1.

**Table I**

| Time (min) | Voltage (v) |
|---|---|
| 1 | 5.09 |
| 50 | 5.09 |
| 100 | 5.19 |
| 150 | 5.42 |
| 200 | 5.57 |
| 250 | 6.27 |
| 270 | 7.03 |
| 300 | 8.63 |
| 310 | 9.62 |
| 320 | 10.14 |
| 330 | 8.94 |
| 340 | 8.19 |

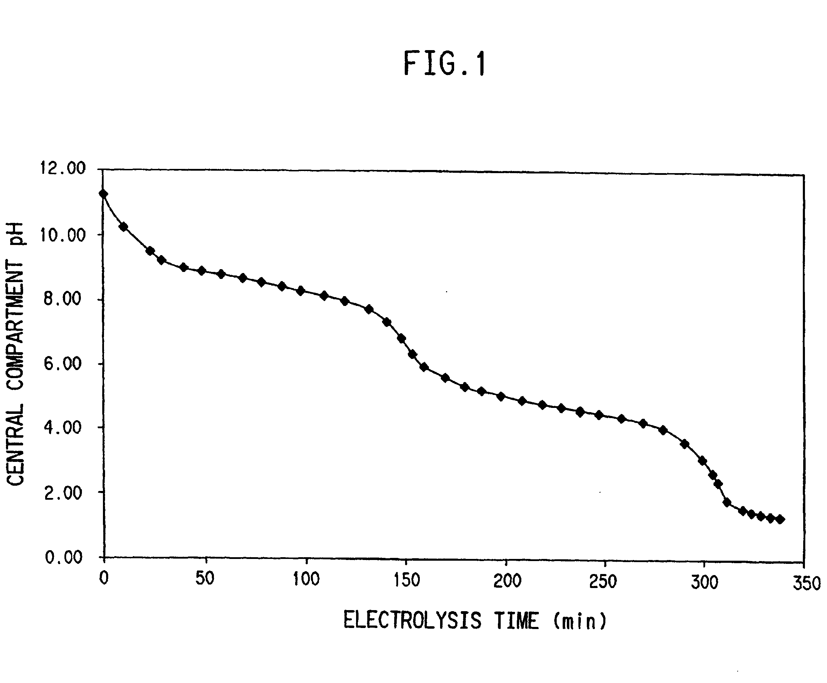

Figure 1 shows the correlation of the pH of the center compartment solution with electrolysis time. It is believed that the inflection point at about 150 min represents the point at which z is approximately 1.0, or the compound present in solution is approximately the monopotassium salt of PHBA. As the time of electrolysis approaches 300 min it is believed that z is becoming quite small, so that at perhaps about 320 min the solute in the center compartment is almost pure PHBA.

### COMPARATIVE EXAMPLE 1

The apparatus used was similar to that in Example 1 except the PHBA solution reservoir was an open Erlenmeyer flask on a hot plate, and there was no filter or auxiliary heater in the PHBA solution lines.

A solution of the dipotassium salt of p-hydroxybenzoic acid (PHBA) was made by dissolving 120 g of PHBA and 114.6 g of KOH (pellets nominally containing 85 weight percent KOH, 15% water) in 400 mL of water. This was placed in the center compartment of the cell, and the solution in all three compartments were separately circulated. The electrolysis was started and continued so that the voltage was varied to maintain a constant current of 15 A (ampere). During the electrodialysis water was added as necessary to replace evaporative losses.

The voltages required vs. the electrolysis time elapsed for selected times during the electrolysis are shown Table 2.

**Table 2**

| Time | Voltage | Remarks |
|---|---|---|
| 3 | 5.65 | |
| 70 | 5.82 | |
| 78 | 5.83 | |
| 120 | 6.43 | |
| 125 | 7.05 | |
| 130 | 8.03 | |
| 135 | 9.96 | |
| 140 | 10.7 | Gas bubbles at anode |
| 145 | 11.8 | |
| 150 | 12.2 | |
| 155 | 13.1 | |
| 160 | 13.9 | |
| 165 | 14.5 | Heat off to center compartment |
| 180 | 15.4 | Crystals forming in PHBA solution |
| 240 | 20.4 | PHBA inlet line plugged |
| 255 | 19.6 | Crystals at PHBA solution surface |
| 261 | 50.0+ | PHBA inlet line plugged |

It is clear that before complete electrolysis of the potassium salt of PHBA could be accomplished the cell required excessive voltage to operate, and in fact plugged with crystals that had formed in the PHBA (and/or its potassium salt) solution.

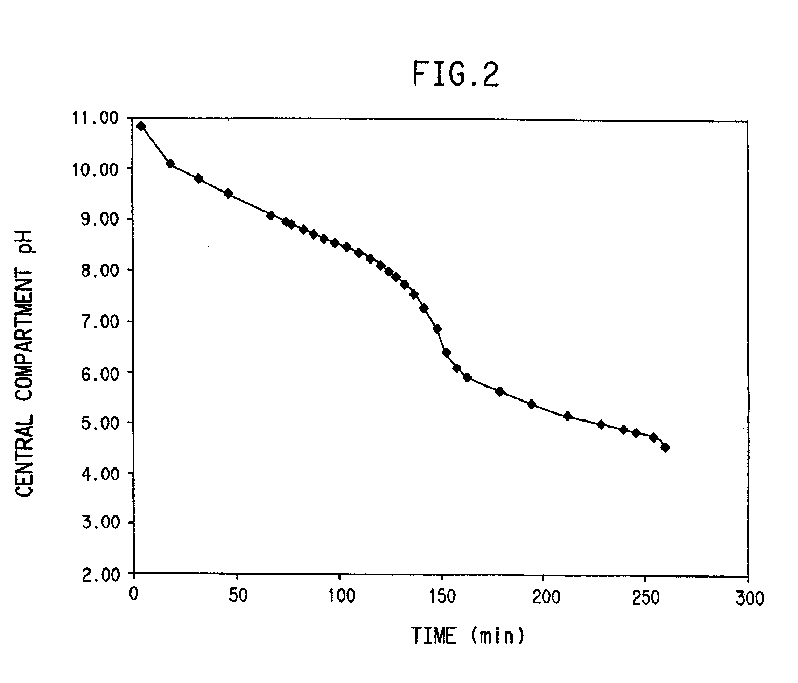

Figure 2 shows the correlation of the pH of the center compartment solution with electrolysis time. It is believed that the inflection point at about 150 min represents the point at which y is approximately 1.0, or the compound present in solution is approximately the monopotassium salt of PHBA. As the time of electrolysis went over 250 min, it is believed that y was approaching 2.0.

## Claims

1. A process for the preparation of p-hydroxybenzoic acid from its alkali metal salt, comprising, electrodialyzing an aqueous solution of a first compound of the formula (OR¹CO₂)HₜM₂₋ₜ to produce a second compound of the formula (OR¹CO₂)H_{y}M_{2-y} and MOH, wherein:
R¹ is p-phenylene;
t is zero to 1.50;
M is an alkali metal cation; and
y is 1.95 to 2.00;
and provided that said electrodialysis is carried out at a temperature of 75°C or more.

2. The process as recited in Claim 1 wherein said alkali metal cation is potassium.

3. The process recited in Claim 2 wherein in said second compound y is 1.97 or more.

4. The process as recited in Claim 2 wherein t is 0 to 0.5.

5. The process as recited in Claim 2 wherein t is 0.0.

6. The process as recited in Claim 1 wherein said temperature is 80°C to an atmospheric boiling point of said aqueous solution.

7. The process as recited in Claim 2 wherein said temperature is 85°C to an atmospheric boiling point of said aqueous solution.

8. The process as recited in claim 2 wherein a concentration of free p-hydroxybenzoic acid equivalent is 13 to 35 percent by weight.

9. The process as recited in Claim 2 wherein a concentration of free p-hydroxybenzoic acid equivalent is 15 to 30 percent by weight.

10. The process as recited in Claim 1 which is carried out in a 3 compartment cell.

## Patentansprüche

1. Verfahren für die Herstellung von p-Hydroxybenzoesäure aus ihrem Alkalimetalsalz, umfassend das Elektrodialysieren einer wässrigen Lösung einer ersten Verbindung der Formel (OR¹CO₂)HₜM₂₋ₜ zur Herstellung einer zweiten Verbindung der Formel (OR¹CO₂)H_{y}M_{2-y} und MOH, wobei:
R¹ p-Phenylen ist;
t null bis 1,50 beträgt;
M ein Alkalimetallkation ist; und
y 1,95 bis 2,00 beträgt;
und vorausgesetzt, dass die Elektrodialysierung bei einer Temperatur von 75°C oder mehr durchgeführt wird.

2. Verfahren nach Anspruch 1, worin das Alkalimetallkation kalium ist.

3. Verfahren nach Anspruch 2, worin in der zweiten Verbindung y 1,97 oder mehr beträgt.

4. Verfahren nach Anspruch 2, worin t 0 bis 0,5 beträgt.

5. Verfahren nach Anspruch 2, worin t 0,0 beträgt.

6. Verfahren nach Anspruch 1, worin die Temperatur von 80°C bis zu einem atmosphärischen Siedepunkt der wässrigen Lösung beträgt.

7. Verfahren nach Anspruch 2, worin die Temperatur von 85°C bis zu einem atmosphärischen Siedepunkt der wässrigen Lösung beträgt.

8. Verfahren nach Anspruch 2, worin eine Konzentration des freien p-Hydroxybenzoesäureäquivalents 13 bis 35 Gew.% beträgt.

9. Verfahren nach Anspruch 2, worin eine Konzentration des freien p-Hydroxybenzoesäureäquivalents 15 bis 30 Gew.% beträgt.

10. Verfahren nach Anspruch 1, das in einer Zelle mit 3 Kammern durchgeführt wird.

## Revendications

1. Procédé pour la préparation d'un acide p-hydroxybenzoïque à partir de son sel de métal alcalin, comprenant l'électrodialyse d'une solution aqueuse d'un premier composé de la formule (OR¹CO₂)HₜM₂₋ₜ pour produire un deuxième composé de la formule (OR¹CO₂)H_{y}M_{2-y} et MOH, où:
R₁ est du p-phénylène;
t est compris entre zéro et 1,50;
M est un cation de métal alcalin; et
y est compris entre 1,95 à 2,00;
et à condition que ladite électrodialyse soit exécutée à une température de 75°C ou plus.

2. Procédé selon la revendication 1, où ledit cation de métal alcalin est du potassium.

3. Procédé selon la revendication 2, où ledit deuxième composé y s'élève à 1,97 ou plus.

4. Procédé selon la revendication 2, où t est compris entre 0 et 0,5.

5. Procédé selon la revendication 2, où t s'élève à 0,0.

6. Procédé selon la revendication 1, où ladite température va de 80°C jusqu'à un point d'ébullition atmosphérique de ladite solution aqueuse.

7. Procédé selon la revendication 2, où ladite température va de 85°C jusqu'à un point d'ébullition atmosphérique de ladite solution aqueuse.

8. Procédé selon la revendication 2, où une concentration d'équivalent d'acide p-hydroxybenzoïque libre est comprise entre 13 et 35% en poids.

9. Procédé selon la revendication 2, où une concentration d'équivalent d'acide p-hydroxybenzoïque est comprise entre 15 et 30% en poids.

10. Procédé suivant la revendication 1, qui est exécuté dans une cellule à 3 compartiments.
